# EUROPÄISCHE PATENTSCHRIFT

(11) **EP 0 562 509 B1**
(45) Veröffentlichungstag und Bekanntmachung des Hinweises auf die Patenterteilung: **21.08.1996**
(21) Anmeldenummer: 93104653.6
(22) Anmeldetag: 22.03.1993
(51) Int. Cl.: B01J 21/20, B01J 21/18, C07C 17/08, C07C 19/08, C01B 31/08

(54) **Verfahren zur Reaktivierung eines bei der Herstellung von 1,1,1,2,3,3,3-Heptafluorpropan (R 227) eingesetzten Aktivkohle-Katalysators**
Reactivation of an activated carbon catalyst used for preparing 1,1,1,2,3,3,3-heptafluoropropane
Procédé de réactivation d'un catalyseur en charbon actif employé pour la préparation du 1,1,1,2,3,3,3-heptafluoropropane

(30) Priorität: 26.03.1992 DE 4209800
(43) Veröffentlichungstag der Anmeldung: 29.09.1993
(73) Patentinhaber: HOECHST AKTIENGESELLSCHAFT, 65926 Frankfurt am Main (DE)
(72) Erfinder: Hopp, Peter, Dr., W-6238 Hofheim am Ts. (DE); Wirth, Uwe, Dr., W-6451 Mainhausen (DE)

(56) Entgegenhaltungen:
- EP-A- 0 418 573
- DD-A- 215 921
- DE-A- 2 641 429
- US-A- 3 714 336
- US-A- 4 347 156

## Beschreibung

Als ein Ersatzstoff für die ozongefährdenden vollhalogenierten Fluorchlorkohlenwasserstoffe ist in einigen Anwendungsfällen 1,1,1,2,3,3,3-Heptafluorpropan (R 227) vorgesehen. Bei seiner Herstellung aus Hexafluorpropen und HF benutzt man als Katalysator Aktivkohle (GB-PS 902590). Es wurde jedoch festgestellt, daß der Katalysator schon nach relativ kurzer Zeit seine Aktivität verliert. Der Aktivitätsverlust äußert sich unter anderem darin, daß der Hot spot, der zu Beginn der Umsetzung am Anfang der Katalysatorschüttung (Eintrittsstelle von Hexafluorpropen und HF) liegt, allmählich an das Ende der Schüttung wandert. Gleichzeitig stellte man eine Gewichtszunahme der Aktivkohle fest. Es stellte sich die Aufgabe, ein Verfahren zu entwickeln, das eine Reaktivierung des Kohlekatalysators ermöglicht.

Es ist bereits bekannt, daß man bei Aktivkohle, die als Adsorptionsmittel benutzt wurde, durch Temperaturerhöhung manchmal eine Desorption der adsorbierten Stoffe und damit eine Regeneration des Adsorptionsmittel erreichen kann (Ullmann's Encycl., 4. Auflage, Band 2 (1972) S. 610 - 613). Dabei werden im allgemeinen Temperaturen von etwa 300-500°C angewandt. Bei mangelnder Flüchtigkeit des Adsorbats tritt jedoch beim Erhitzen keine Regenerierung ein. Stattdessen verkoken die adsorbierten organischen Verunreinigungen beim Erhitzen und verursachen eine bleibende Schädigung. In diesem Fall kann man durch Behandlung mit CO₂- und wasserdampfhaltigen Gas bei etwa 900 °C wieder neue Adsorptionsporen schaffen. Diese Reaktivierung der Aktivkohle als Adsorptionsmittel ist jedoch mit Kohle-Verlust verbunden, da eine chemische Reaktion der Aktivkohle mit dem Reaktivierungsgas stattfindet (Ullmann, loc. cit., insbesondere S. 612).

Überraschenderweise wurde nun gefunden, daß man Aktivkohle, die als Katalysator bei der Herstellung von R 227 eingesetzt wurde und dabei ihre katalytische Aktivität ganz oder teilweise verloren hat, durch Erhitzung auf 450 bis 900 °C in einem Inertgasstrom oder im Vakuum reaktivieren kann. Dies ist überraschend, da zu erwarten war, daß die Aktivkohle durch Adsorption von nichtflüchtigen Oligomeren inaktiviert ist, die bei Erhitzung auf 450-900°C verkoken würden.

Gegenstand der Erfindung ist daher ein Verfahren zur Reaktivierung eines bei der Herstellung von 1,1,1,2,3,3,3-Heptafluorpropan (R 227) eingesetzten Aktivkohle-Katalysators, dadurch gekennzeichnet, daß man den Katalysator in einem Inertgasstrom oder bei vermindertem Druck auf 450 bis 900 °C erhitzt.

Vorzugsweise wird im Inertgasstrom gearbeitet. Als Inertgas wird vorzugsweise Stickstoff eingesetzt. Das Inertgas kann auf 450 bis 900 °C erhitzt werden; in diesem Fall ist eine zusätzliche Erhitzung der Aktivkohle mit anderen Mitteln nicht notwendig, wenngleich möglich. Wenn das Inertgas jedoch nicht selbst auf 450 bis 900 °C erhitzt wird, muß die Aktivkohle mit anderen Mitteln auf die genannte Temperatur erhitzt werden; vorzugsweise wird sie mit Mikrowellen bestrahlt. Der Inertgasstrom bzw. die Druckverminderung bewirkt den Abtransport der bei der Erhitzung der Aktivkohle gebildeten Gase.

Vorzugsweise wird der Aktivkohle-Katalysator auf 550 bis 800 °C, insbesondere auf 600 bis 800 °C erhitzt.

Nach 1 bis 24-stündiger, vorzugsweise 5 bis 10-stündiger Behandlung ist die Reaktivierung abgeschlosssen. Diese Behandlung kann innerhalb oder außerhalb der Produktionsanlage für das R 227 stattfinden. Bei Erhitzung mit Hilfe von Mikrowellen läuft die Reaktivierung besonders schnell ab, sogar bereits bei Temperaturen von 450 bis 650 °C.

Daß die Reaktivierung im wesentlichen vollständig ist, erkennt man daran, daß der Hot spot wieder an die Eintrittsstelle von Hexafluorpropen und HF in die Katalysatorschüttung gewandert ist.

Falls bei vermindertem Druck gearbeitet wird, beträgt dieser vorzugsweise weniger als 1 mbar.

Man kann auch überhitzten Wasserdampf im Gemisch mit CO₂ bei 450 bis 900 °C verwenden.

Die folgenden Beispiele dienen zur Erläuterung der Erfindung.

### Beispiel 1

In einen Rohrreaktor aus Quarzglas wurden 30 g Aktivkohle eingefüllt, die in der Synthese von R 227 aus Hexafluorpropen und HF als Katalysator benutzt worden war und dabei ihre Aktivität weitgehend verloren hatte. Der Rohrreaktor wurde in eine Mikrowellenapparatur eingebracht. Die im Reaktor befindliche Aktivkohle wurde 20 Minuten lang mit Mikrowellen bei einem Leistungseintrag von 400 Watt und einer Frequenz von 2450 MHz bestrahlt, wobei sich die Aktivkohle auf 600 °C erhitzte. Während der Erhitzung wurde ein Stickstoffstrom von 10 l/h über die Aktivkohle geleitet. Anschließend hatte sie wieder ihre volle katalytische Aktivität zurückgewonnen, was man an der Lage des Hot spot erkennt.

### Beispiel 2

In einem elektrisch beheizten Ofen wurden 3 kg Aktivkohle, die in der R 227-Synthese benutzt worden war und ihre katalytische Aktivität weitgehend verloren hatte, innerhalb von 5 Stunden auf 300 °C und innerhalb weiterer 10 Stunden auf 550 °C erhitzt. Diese Temperatur wurde 9 Stunden aufrechterhalten. Während der gesamten Zeit wurde die Aktivkohle mit 50 l/h Stickstoff gespült. Anschließend hatte sie ihre volle katalytische Aktivität zurückgewonnen.

## Patentansprüche

1. Verfahren zur Reaktivierung eines bei der Herstellung von 1,1,1,2,3,3,3-Heptafluorpropan (R 227) eingesetzten Aktivkohle-Katalysators, dadurch gekennzeichnet, daß man den Katalysator in einem Inertgasstrom oder bei vermindertem Druck auf 450 bis 900 °C erhitzt.

2. Verfahren nach Anspruch 1, dadurch gekennzeichnet, daß man den Katalysator auf 550 bis 800 °C erhitzt.

3. Verfahren nach Anspruch 1, dadurch gekennzeichnet, daß man den Katalysator auf 600 bis 800 °C erhitzt.

4. Verfahren nach einem der Ansprüche 1 bis 3, dadurch gekennzeichnet, daß man den Katalysator durch Mikrowellen-Bestrahlung erhitzt.

5. Verfahren nach einem der Ansprüche 1 bis 4, dadurch gekennzeichnet, daß man in einem Inertgasstrom arbeitet.

6. Verfahren nach Anspruch 5, dadurch gekennzeichnet, daß man Stickstoff als Inertgas einsetzt.

## Claims

1. A process for the reactivation of an activated charcoal catalyst employed in the preparation of 1,1,1,2,3,3,3-heptafluoropropane (R 227), which comprises heating the catalyst to from 450 to 900°C in a stream of inert gas or under reduced pressure.

2. The process as claimed in claim 1, wherein the catalyst is heated to from 550 to 800°C.

3. The process as claimed in claim 1, wherein the catalyst is heated to from 600 to 800°C.

4. The process as claimed in any one of claims 1 to 3, wherein the catalyst is heated by microwave irradiation.

5. The process as claimed in any one of claims 1 to 4, wherein a stream of inert gas is used.

6. The process as claimed in claim 5, wherein the inert gas employed is nitrogen.

## Revendications

1. Procédé pour la réactivation d'un catalyseur de charbon actif utilisé dans la préparation du 1,1,1,2,3,3,3-heptafluoropropane (R227), caractérisé en ce qu'on chauffe le catalyseur dans un courant de gaz inerte ou sous une pression réduite à 450 à 900°C.

2. Procédé selon la revendication 1, caractérisé en ce qu'on chauffe le catalyseur à 550 à 800°C.

3. Procédé selon la revendication 1, caractérisé en ce qu'on chauffe le catalyseur à 600 à 800°C.

4. Procédé selon l'une des revendications 1 à 3, caractérisé en ce qu'on chauffe le catalyseur par irradiation par des micro-ondes.

5. Procédé selon l'une des revendications 1 à 4, caractérisé en ce qu'on travaille dans un courant de gaz inerte.

6. Procédé selon la revendication 5, caractérisé en ce qu'on utilise l'azote comme gaz inerte.
